# DEMANDE DE BREVET EUROPEEN

(11) **EP 0 945 101 A1**
(43) Date de publication de la demande: **29.09.1999**
(21) Numéro de dépôt: 99870045.4
(22) Date de dépôt: 12.03.1999
(51) Int. Cl.: A61B 5/087

(54) **Réducteur de pression pour mesure du débit expiratoire sous NEP**

(30) Priorité: 26.03.1998 BE 9800239
(71) Demandeur: Martinot, Guy, 5540 Hastière (BE)
(72) Inventeur: Martinot, Guy, 5540 Hastière (BE)
(74) Mandataire: Kuborn, Jacques

(57) **Abrégé**

Générateur de dépression comprenant un conduit principal (2) présentant une paroi interne conique divergeant vers une extrémité ouverte (3), et une source de gaz sous pression à injecter dans ledit conduit (2) pour y créer une dépression à l'extrémité (19) opposée à ladite extrémité ouverte (3), caractérisé en ce qu'il comprend une chambre (7) disposée entre la source de gaz sous pression et le conduit principal (2), communiquant avec le conduit principal (2) par un passage périphérique (8) configuré pour diriger le courant de gaz comprimé en expansion vers ladite extrémité (3).

## Description

La présente invention concerne un appareil de mesure du degré de limitation du débit expiratoire sous NEP ("negative expiratory pressure") chez des sujets au repos et à l'effort. Cette mesure demande la création d'une dépression à travers un pneumotachographe, et ne demande aucune collaboration du sujet examiné.

La méthode sous NEP est décrite par exemple dans l'article "50 ans d'expiration forcée" publié dans la Revue des Maladies Respiratoires, 1997, 14 pages 431-443; dans l'article "A simple method to monitor performance of forced vital capacity" the American Physiological Society, pages 693 et suivantes (référence bibliographique 0161-7567/96) ; dans l'article "Relationship between chronic dispnea and expiratory flow limitation in patient with COPD" American Journal of Respiratory and Critical Care Medecine, vol 154, 1996, pages 1726-1734. Ces publications sont incorporées ici à titre de référence.

Selon ces publications, le pneumotachographe est relié à un Venturi ou à un dispositif aspirant, et permet de comparer un enregistrement de la courbe de ventilation débit-volume expiratoire avec et sans le NEP.

Pour donner des mesures précises et fiables, ce type d'appareil doit présenter diverses caractéristiques, parmi lesquelles: une résistance à l'écoulement négligeable, et un écoulement essentiellement laminaire, pour éviter de modifier les conditions naturelles de ventilation; des caractéristiques d'écoulement indépendantes du sens de l'écoulement (courant d'inspiration et d'expiration); un temps d'établissement de la dépression aussi court que possible, et avec le moins de perturbation possible, pour éviter des phénomènes du type "overshoot", avec création d'un pic initial de dépression, et donc un pic correspondant dans le débit; un "volume mort" (volume du conduit interne du dispositif) aussi faible que possible, pour éviter que le sujet ne ré-inspire l'air qu'il a expiré au cycle précédent, ce qui peut entraîner une hyperventilation; et enfin un poids aussi faible que possible puisque le sujet doit, lors du test à l'effort, maintenir l'appareillage avec sa bouche, et qu'un poids important peu poser des problèmes à cet égard.

Selon l'invention, on se propose de fournir, pour ce type d'appareillage, un dispositif générateur de dépression présentant les qualités requises.

Un objet de l'invention est donc de fournir un générateur de dépression pour fluide gazeux, comprenant un conduit principal présentant une paroi interne conique divergeant vers une extrémité ouverte, et une source de gaz sous pression à injecter dans ledit conduit pour y créer une dépression à l'extrémité opposée à ladite extrémité ouverte, caractérisé en ce qu'il comprend une chambre disposée entre la source de gaz sous pression et le conduit principal, communiquant avec le conduit principal par un passage périphérique configuré pour diriger le courant de gaz comprimé en expansion vers ladite extrémité.

Selon d'autres caractéristiques, le générateur de dépression selon l'invention comprend un manchon femelle assemblé au conduit principal, et ledit passage est constitué par un interstice entre l'extrémité interne du conduit principal et la paroi interne du manchon; la paroi interne du manchon comprend un ou deux épaulements, ou encore un tronçon courbe constituant injecteur, pour canaliser le courant de gaz sous pression vers l'extrémité ouverte du conduit principal; la partie du conduit principal située entre le passage et l'extrémité ouverture a une longueur suffisante pour assurer un écoulement sensiblement laminaire du courant de gaz comprimé.

D'autres aspects, caractéristiques et avantages de l'invention apparaîtront de la description qui suit, et du dessin annexé sur lequel
La figure 1 est une vue en coupe d'un mode de réalisation d'un générateur de dépression selon l'invention, à l'état désassemblé,
La figure 2 est une vue en coupe du mode de réalisation de la figure 1, à l'état assemblé,
Les figures 3 et 4 sont des vues de détail de variantes du mode de réalisation des figures 1 et 2.

En se reportant au dessin, le générateur de dépression 1 comprend un conduit 2 ouvert à une extrémité 3 et assemblé par son extrémité opposée à un manchon creux, femelle, coaxial 4.

Le conduit 2 et le manchon 4 sont assemblés par exemple par des filetages 5, 5' coopérants.

Le manchon 4 est d'autre part percé d'un orifice 6 prévu pour recevoir le raccordement d'une tuyau d'alimentation en gaz comprimé destiné à créer la dépression.

Dans sa portion logée à l'intérieur du manchon 4, le conduit 2 présente dans sa paroi externe un évidement périphérique délimitant une chambre 7 avec le paroi interne opposée du manchon 4.

Comme on le voit au dessin, cette chambre 7 débouche dans l'espace interne du conduit et du manchon par un chemin 8 défini par un interstice périphérique entre la paroi interne du manchon 4, se terminant pas des épaulements 9, 9', et un épaulement externe 14 du conduit 2; la paroi interne du conduit 2 est d'autre part usinée avec une conicité s'évasant vers l'extrémité ouverte 3, et se raccorde par un congé 10 à la paroi externe, de manière à favoriser le passage de l'air comprimé de la chambre 7 vers l'extrémité 3, pour créer une dépression à l'extrémité 19 opposée, prévue pour être reliée à un pneumotachographe 11 ou appareil analogue (ébauché en pointillé).

L'orifice 6 est relié par un tuyau à une source d'air comprimé (non représentée), par l'intermédiaire d'une vanne électromagnétique 13 asservie.

Lorsqu'une dépression doit être créée, une commande (non représentée) agit sur la vanne 13 pour admettre l'air comprimé qui est alors envoyé dans la chambre 7, et de là dans le conduit ou tuyère conique 2, en direction de l'extrémité ouverture 3, pour créer une dépression à l'extrémité opposée 19.

Le but des épaulements 9, 9' du manchon 4, ainsi que de l'épaulement 14 de la chambre 7, est de canaliser l'air comprimé venant de la conduite 7 en direction de l'extrémité 3.

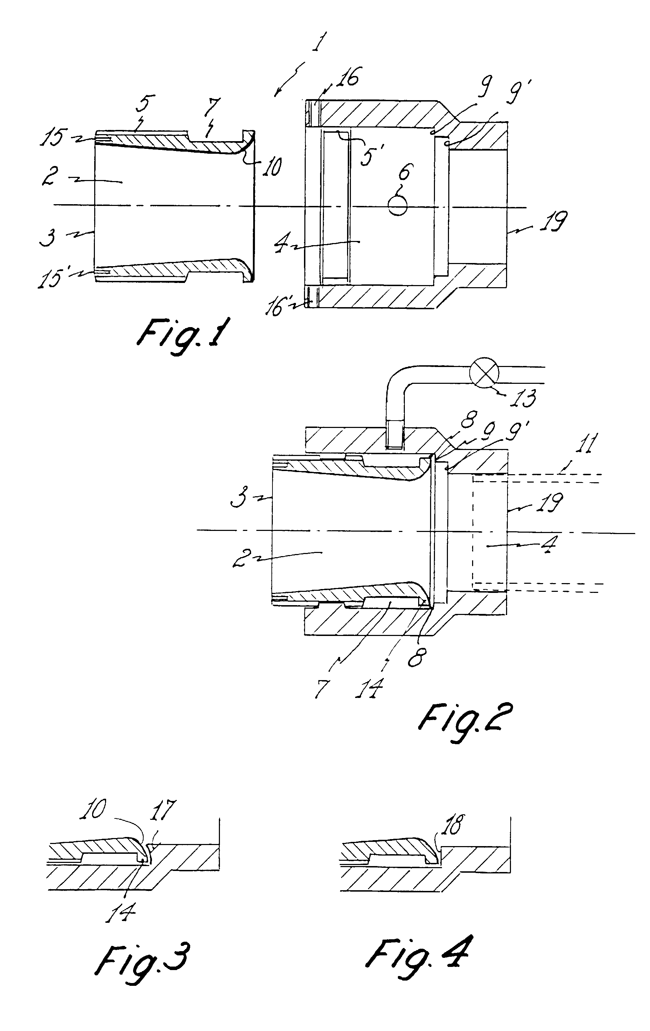

Dans la variante représentée à la figure 3, les épaulements 9, 9' sont remplacés par un tronçon de paroi courbe 17 définissant un injecteur avec le congé 10 opposé.

L'avantage essentiel des épaulements 9, 9' par rapport à la paroi courbe 17 est essentiellement la facilité de l'usinage de la pièce.

La variante de la figure 4 comprend un seul épaulement 18. Ce mode de réalisation est cependant moins favorable en ce sens que, toutes choses étant égales, il ne permet d'atteindre qu'une dépression inférieure à celle atteinte avec les modes de réalisation des figures 1 à 3.

Le but de la chambre 7 est de servir en quelque sorte d'organe tampon permettant d'obtenir une dépression calibrée extrêmement stable, et sans pic d'établissement (overshoot), permettant ainsi d'obtenir des mesures de débit expiratoire fiables et reproductibles.

Comme on le comprendra, la valeur de la dépression obtenue du côté de l'extrémité 19 dépendra de la pression de l'air comprimé admis par l'orifice 6, ainsi que des dimensions du passage 8.

Pour une pression d'admission donnée de la source d'air comprimé, le calibrage du dispositif à une dépression déterminée peut se faire simplement en obturant l'ouverture 19, et en mesurant la dépression à l'aide d'une colonne manométrique, tout en faisant tourner le conduit 2 par rapport au manchon 4, par exemple à l'aide d'une pince s'engageant dans des logements 15, 15', jusqu'à ce que la dépression mesurée atteigne la valeur choisie.

La position relative du conduit et du manchon peut alors être bloquée par exemple à l'aide de vis de blocage insérées dans des alésages 16, 16' dans le manchon.

Pour un diamètre interne de ± 28 mm à l'extrémité 19 prévue pour être raccordée à un pneumotachographe ou analogue, on atteint selon l'invention une longueur hors tout de l'ordre de 60 à 65 mm, ce qui donne un volume mort très favorable.

Bien entendu, l'invention n'est pas limité au mode de réalisation représenté et décrit, qui n'a été choisi qu'à titre d'exemple.

## Revendications

1. Générateur de dépression pour fluide gazeux, comprenant un conduit principal (2) présentant une paroi interne conique divergeant vers une extrémité ouverte (3), et une source de gaz sous pression à injecter dans ledit conduit (2) pour y créer une dépression à l'extrémité (19) opposée à ladite extrémité ouverte (3),
caractérisé en ce qu'il comprend
- une chambre (7) disposée entre la source de gaz sous pression et le conduit principal (2),
- communiquant avec le conduit principal (2) par un passage périphérique (8) configuré pour diriger le courant de gaz comprimé en expansion vers ladite extrémité (3).

2. Générateur de dépression selon la revendication 1, caractérisé en ce qu'il comprend un manchon femelle (4) assemblé au conduit principal (2), et en ce que ledit passage (8) est constitué par un interstice entre l'extrémité interne (14) du conduit principal (2) et la paroi interne (9, 9'; 17; 18) du manchon (4).

3. Générateur de dépression selon la revendication 1 ou 2, caractérisé en ce que la paroi interne du manchon (4) comprend deux épaulements (9, 9') destinés à canaliser le courant de gaz sous pression vers l'extrémité ouverte (3) du conduit principal (2).

4. Générateur de dépression selon la revendication 1 ou 2, caractérisé en ce que la paroi interne du manchon (4) comprend un tronçon de paroi courbe (17) formant injecteur avec la paroi (10) opposée du conduit (2), pour canaliser le courant de gaz sous pression vers l'extrémité ouverte (3) du conduit principal (2).

5. Générateur de dépression selon la revendication 1 ou 2, caractérisé en ce que la paroi interne du manchon (4) comprend un seul épaulement (18) destiné à canaliser le courant de gaz sous pression vers l'extrémité ouverte (3) du conduit principal (2).

6. Générateur de dépression selon l'une quelconque des revendications précédentes, caractérisé en ce que la partie du conduit principal (2) située entre le passage (8) et l'extrémité ouverture (3) a une longueur suffisante pour assurer un écoulement sensiblement laminaire du courant de gaz comprimé (4).
